# EUROPÄISCHE PATENTANMELDUNG

(11) **EP 4 663 209 A1**
(43) Veröffentlichungstag der Anmeldung: **17.12.2025**
(21) Anmeldenummer: 24181260.1
(22) Anmeldetag: 10.06.2024
(51) Int. Cl.: A61L 2/20, A61L 2/08, A61L 2/24, B65B 55/08

(54) **VERFAHREN ZUM SERIELLEN TRANSFER VON OBJEKTEN DURCH EINE PROZESSKAMMER ZUR WEITERVERARBEITUNG IN EINEM CONTAINMENT UNTER ASEPTISCHEN BEDINGUNGEN UND PRODUKTIONSANLAGE DAZU**

(71) Anmelder: SKAN Stein AG, 4332 Stein (CH)
(72) Erfinder: ALAVA, Andreas, 4055 Basel (CH); BERG, Felix Anton, 5080 Laufenburg (CH); BIELMANN, Michael, 8800 Thalwil (CH); HOLZER, Manfred, 79576 Weil am Rhein (DE); LEHMANN, Frank Martin, 4102 Binningen (CH); SEURET, Dominique, 4132 Muttenz (CH)
(74) Vertreter: Ullrich, Gerhard

(57) **Zusammenfassung**

Das erfindungsgemässe Verfahren mit dazu konzipierter Produktionsanlage (**1**) ist zum seriellen Transfer von Objekten (**O1-Ox**) durch eine Prozesskammer (**5**) zur Weiterverarbeitung in einem Containment (**6**) unter aseptischen Bedingungen bestimmt. Der Prozesskammer (**5**) ist eine Bereitstellungszone (**2**) vorgelagert, aus der das jeweilige Objekt (**O1-Ox**) in die Prozesskammer (**5**) geladen wird, um hier die Oberfläche des jeweiligen Objekts (**O1-Ox**) zu dekontaminieren. Die Bereitstellungszone (**2**) mit der Prozesskammer (**5**) und dem sich dran anschliessenden Containment (**6**) bilden eine in einem Aufstellungsraum (**A**) errichtete Produktionsanlage (**1**). In der Prozesskammer (**5**) sind zumindest zwei Elektronen-Strahlungsquellen (**E1,E2**) installiert, zwischen denen das jeweilige Objekt (**O1-Ox**) zur Erzielung seiner äusserlichen Dekontamination hindurchgeführt wird. Die Objekte (**O1-Ox**) haben z.B. die äussere Gestalt wannenförmiger Tubs oder Trays, und darin gelagerte Artikel sind z.B. pharmazeutische Phiolen, Vials, Spritzen oder Karpulen.

## Beschreibung

### Anwendungsgebiet der Erfindung

Die vorliegende Erfindung betrifft ein Verfahren zum seriellen Transfer von Objekten durch eine Prozesskammer zur Weiterverarbeitung in einem Containment unter aseptischen Bedingungen. Der Prozesskammer ist eine Bereitstellungszone vorgelagert, aus der das jeweilige Objekt in die Prozesskammer geladen wird, um hier die Oberfläche des jeweiligen Objekts zu dekontaminieren. Die Bereitstellungszone mit der Prozesskammer und dem sich dran anschliessenden Containment bilden eine in einem Aufstellungsraum errichtete Produktionsanlage. In der Prozesskammer sind zumindest zwei Elektronen-Strahlungsquellen installiert, zwischen denen das jeweilige Objekt zwecks äusserlicher Dekontamination hindurchgeführt wird. Die Objekte haben z.B. die äussere Gestalt wannenförmiger Tubs, und darin gelagerte Artikel sind z.B. pharmazeutische Phiolen, Vials, Spritzen oder Karpulen. Ein weiterer Gegenstand der Erfindung ist eine zur Durchführung des Verfahrens konzipierte Produktionsanlage.

Soweit für die vorliegende Erfindung der Begriff "Dekontamination" als Substantiv in jeder deklinierten Weise, als Verb in jeder konjugierten Weise oder in adjektivischer Form verwendet wird, schliesst dies allein zur sprachlichen Verkürzung auch den chemisch und biologisch technisch differenziert definierten Begriff "Sterilisation" und dessen Verb und Adjektiv ein.

### Stand der Technik

Es ist bekannt, zur Dekontamination und Sterilisation von Oberflächen in der Pharma- und Verpackungsindustrie niederenergetische Elektronenbestrahlung einzusetzen. Derartige Einrichtungen lassen sich beim Anwender mit relativ geringem apparativem Aufwand installieren und betreiben. Anlagen mit Gamma- oder Hochenergie-Elektronenstrahlen hingegen erfordern substanzielle Aufbauten und eignen sich daher nur für engere Anwenderkreise, wie z.B. zentrale Dienstleister. Anlagen zum Betrieb mit niederenergetischen Elektronen weisen Linearkathoden oder Punktquellen mit nachfolgender elektromagnetischer Aufweitung, sogenannte Scanner, auf. Eine Sterilisationsanlage mit einer Linearkathode ist z.B. in der US 8,772,743 B2 offenbart. Eine Linearkathode besteht im Wesentlichen aus einem Filament, welches als Elektronenquelle wirkt und in einer Kathode auf negativem Potential gehalten wird. Das Filament ist in einem Vakuumbehältnis angeordnet, das ein aus dünner Folie beschaffenes Elektronenaustrittsfenster aufweist. Die Elektronen werden von der Kathode in Richtung Elektronenaustrittsfenster beschleunigt und gewinnen somit genügend kinetische Energie, dieses zu durchdringen, wobei das Vakuum innerhalb des Behältnisses erhalten bleibt. Daher lässt sich der Elektronenstrahl auch bei Umgebungsdruck für bestimmte Anwendungen industriell nutzen. So lässt sich ein Behältnis, dessen Oberfläche zu dekontaminieren ist, in einer Prozesskammer durch eine Elektronenwolke in einer Translationsbewegung transportieren, welche dadurch das Behältnis allseits bestrahlt. Dabei umfassen die Anlagen in der Regel mehrere Strahlungsquellen, wie z.B. mit zwei Strahlungsquellen in der US 2012/0 217 042 A1 beschrieben.

Auch beim Ein- und Ausschleusen der zu behandelnden Behältnisse in die Prozesskammer darf von der durch die Elektronenwolke erzeugten Röntgenstrahlung keine Gefährdung für die äussere Umgebung ausgehen. In den Patentpublikationen EP 2 094 313 B1, EP 2 833 928 B1, Patent EP 1 879 629 B1 und EP 3 479 848 B1 werden hierfür verschiedene Schutzbarrieren bzw. der Strahlungsrichtung ausweichende Förderstrecken vorgeschlagen.

Die Sterilisationsanlage gemäss der EP 1 685 853 B1 beruht auf nur einer Strahlungsquelle mit klarer Richtungsausprägung der erzeugten Elektronenwolke. Die Strahlungsbehandlung der gesamten Oberfläche des Behältnisses erreicht man dadurch, dass das Behältnis in einer sequenziellen Abfolge von Rotations- und Translationsbewegungen der Elektronenwolke ausgesetzt wird, dennoch verbleiben mehrere Unzulänglichkeiten. Durch die Strukturierung des Bewegungsablaufs des Behältnisses durch die Elektronenwolke in mehreren sequenziellen Etappen, ergibt sich keine eindeutige Grenze an der Elektronenquelle zwischen sterilem und unsterilem Bereich. Ferner muss auch die Halterung, welche das Behältnis trägt, im Sterilisationsprozess behandelt werden, um eine Rekontamination der am Behältnis gereinigten Oberflächen zu vermeiden, was kaum zuverlässig erreichbar ist. Schliesslich ist bei dem apparativen Aufbau die Überlagerung der Prozesskammer mit einer Laminarströmung aus steriler Luft, wodurch die sterile Prozessseite von der unsterilen Prozessseite getrennt wird, nicht realisierbar.

Die US 10,265,427 B2 hat eine Sterilisationsanlage zum Gegenstand, welche eine Strahlungsquelle, bestehend aus mehreren gerichteten Einzelquellen aufweist, deren Elektronenwolke das zu behandelnde, in sequentiellen Translations- und Rotationsbewegungen geführte Behältnis räumlich umschliesst. Die Abfolge der Sterilisation verschiedener Oberflächenareale des zu behandelnden Behältnisses in räumlich separate Unterprozesse, bietet keine Gewähr dafür, dass lückenlos alle Oberflächenareale und Manipulatoren zum Transport des Behältnisses beim Durchgang durch die Elektronenwolke sterilisiert werden. Ferner stellt sich bei diesem Anlagenkonzept beim Zuführen einer Laminarströmung aus steriler Luft in die Prozesskammer keine klare Grenze zwischen sterilem und unsterilem Bereich ein. Schliesslich erfüllt diese Anlage nicht die Ansprüche eines möglichst geringen gerätetechnischen, kompakten und kosteneffizienten Aufwands.

Aus der WO 2007/107 331 A1 schliesslich ist eine Apparatur, z.B. zur Sterilisation von Pharma- und Medizinalprodukten, bekannt, welche auf zwei keimabtötenden Elektronen-Strahlungsquellen beruht, zwischen denen die zu behandelnden Produkte, bei permanent eingeschalteten Elektronen-Strahlungsquellen, hindurchgeführt werden.

Jüngst wurde von der Anmelderin gemäss Internationaler Patentanmeldung PCT/CH2024/000 003 ein Verfahren und eine zugehörige Produktionsanlage vorgeschlagen, bei welcher der Transport des äusserlich zu dekontaminierenden Objekts durch das Emissionsfeld einer einzigen Elektronen-Strahlungsquelle erfolgt. Eine Fördereinrichtung stützt das aufgenommene Objekt im Wechsel an verschiedenen Oberflächenarealen, sodass die gesamte Oberfläche des Objekts dekontaminiert ist. Hierbei wird der Transport als kombinierte Bewegung des Objekts vor dem Austrittsfenster als kontinuierliche oder schrittweise Vorwärtsbewegung des Objekts und dazu gleichzeitiger Dreh- oder Taumelbewegung um sich selbst oder als schrittweise Vorwärtsbewegung des Objekts und zwischen den vorwärtsweisenden Bewegungsschritten eine Dreh- oder Taumelbewegung um sich selbst, ausgeführt.

### Aufgabe der Erfindung

In Weiterentwicklung zum bisherigen Stand der Technik liegt der Erfindung die Aufgabe zugrunde, ein Verfahren mit zugehöriger Produktionsanlage vorzuschlagen, womit die vollständige Dekontamination der Oberflächen zu behandelnder Objekte mit effizienter Produktivität, geringstmöglichem apparativem Aufwand und hoher Qualität erreicht wird. Hierbei soll in einer Bestrahlungszone innerhalb einer Prozesskammer eine eindeutige stabile Grenze zwischen sterilem und unsterilem Bereich gewahrt bleiben.

### Übersicht über die Erfindung

Das Verfahren ist zum seriellen Transfer von Objekten durch eine Prozesskammer zur Weiterverarbeitung in einem Containment unter aseptischen Bedingungen bestimmt. Der Prozesskammer ist eine Bereitstellungszone vorgelagert, aus der das jeweilige Objekt in die Prozesskammer geladen wird, um hier die Oberfläche des jeweiligen Objekts zu dekontaminieren. Die Bereitstellungszone mit der Prozesskammer und dem sich dran anschliessenden Containment bilden eine in einem Aufstellungsraum errichtete Produktionsanlage. In der Prozesskammer sind zumindest zwei Elektronen-Strahlungsquellen installiert, zwischen denen das jeweilige Objekt zur Erzielung seiner äusserlichen Dekontamination hindurchgeführt wird. Das einzelne Objekt weist eine Aussenkontur auf. Durch eine kontinuierliche Luftführung in der Prozesskammer wird eine Sterilgrenze im Wirkungsbereich einer von den Elektronen-Strahlungsquellen gebildeten Bestrahlungszone gewahrt, und erst ab dieser Sterilgrenze herrschen in Richtung Containment aseptische Verhältnisse. Die zumindest zwei Elektronen-Strahlungsquellen werden nur für eine Zeitdauer zur äusserlichen Bestrahlung des einzelnen dazwischen hindurchgeführten Objekts eingeschaltet, sind aber ansonsten ausgeschaltet.

Die Prozesskammer gliedert sich in die Bestrahlungszone und eine an die Bereitstellungszone angrenzende Vorkammer. Am Übergang von der Bereitstellungszone zur Vorkammer ist eine erste Passage zum Durchlass der Objekte, bei einem geöffneten ersten beweglichen, strahlungsabschirmenden Tor, vorgesehen. Am Übergang von der Bestrahlungszone zum Containment ist eine zweite Passage zum Durchlass der Objekte, bei einem geöffneten zweiten beweglichen, strahlungsabschirmenden Tor, vorgesehen. Am Übergang von der Vorkammer zur Bestrahlungszone ist eine dritte Passage zum Durchlass der Objekte, bei einem geöffneten dritten beweglichen Tor, vorgesehen. Dieses dritte Tor ist in geschlossener Position bei der Luftführung innerhalb der Prozesskammer als Separator partiell luftdurchlässig. Zum beschleunigten Durchsatz des ersten und zweiten Objekts, bei geschlossenem dritten Tor, können das erste und zweite Tor gleichzeitig geöffnet und nach Durchlass beider Objekte durch die jeweilige Passage beide Tore gleichzeitig wieder geschlossen werden.

An der dritten Passage ist ein kragenartiges erstes Strömungselement vorgesehen, das sich in die Vorkammer und/oder in die Bestrahlungszone erstreckt. Das erste Strömungselement ist komplementär zur Aussenkontur des einzelnen gestaltet, um den Luftdurchlass bei geöffnetem drittem Tor und darin einfahrendem bzw. daraus ausfahrendem Objekt zu reduzieren. An der zweiten Passage ist ein kragenartiges zweites Strömungselement vorgesehen, das sich in die Bestrahlungszone und/oder in das Containment erstreckt. Das zweite Strömungselement ist komplementär zur Aussenkontur des einzelnen Objekts gestaltet, um den Luftdurchlass bei geöffnetem zweitem Tor und darin einfahrendem bzw. daraus ausfahrendem Objekt zu reduzieren.

Die zumindest zwei Elektronen-Strahlungsquellen werden im eingeschalteten Betrieb mit unterschiedlicher Beschleunigungsspannung und/oder unterschiedlichem Emissionsstrom betrieben, woraus unterschiedliche Strahlencharakteristiken entstehen, so dass die Wirkung der Elektronen-Strahlungsquellen nicht durch eine das Objekt verschliessende Abdeckung, schädigend in das Innere des Objekts, gelangt.

Alternativ sind die zumindest zwei Elektronen-Strahlungsquellen im eingeschalteten Betrieb in unterschiedlichen Abständen zum jeweils zu bestrahlenden Objekt positioniert, so dass die Elektronenstrahlen der zumindest zwei Elektronen-Strahlungsquellen mit unterschiedlicher Strahlencharakteristik auf das jeweils von den Elektronenstrahlen erfasste Oberflächenareal des Objekts treffen, z.B. damit die Wirkung der Elektronen-Strahlungsquellen nicht durch eine das Objekt verschliessende Abdeckung, schädigend in das Innere des Objekts, gelangt.

Die Zeitdauer der zumindest zwei Elektronen-Strahlungsquellen wird im eingeschalteten Betrieb:
a) vorzugsweise gleichlang und gleichzeitig eingestellt; oder
b) gleichzeitig, aber mit ungleicher Länge eingestellt; oder
c) gleichlang, aber zeitlich versetzt eingestellt; oder
d) mit ungleicher Länge eingestellt.

Das Containment wird von einem aufbereiteten Luftstrom durchströmt, und im Containment herrscht ein erhöhter Überdruck. Die Bestrahlungszone wird ebenfalls von einem aufbereiteten Luftstrom durchströmt, und in der Bestrahlungszone herrscht ein geringer Überdruck. Die Vorkammer wird auch von einem Luftstrom durchströmt, wobei und in der Vorkammer ein Unterdruck herrscht.

Das einzelne Objekt weist ein Behältnis mit einem aseptischen Innenraum auf und hat einen Durchlass, der mit der Abdeckung verschlossen ist, wobei im Innenraum gelagerte Artikel nach Öffnen der Abdeckung zur Behandlung im Containment bestimmt sind.

Das Behältnis ist z.B. ein wannenförmiges Tub oder Tray, wobei:
b) die Abdeckung typischerweise auf dem den Durchlass des Behältnisses umlaufenden Rand versiegelt ist und so den Innenraum des Behältnisses in sterilem Zustand bewahrt;
c) das Behältnis verschiedene Oberflächenareale aufweist, die sich als Konturen zum Unterfassen oder klemmenden Ergreifen des Objekts eignen;
d) die Artikel insbesondere Phiolen, Vials, Spritzen oder Karpulen sind; und
e) von der Abdeckung vorzugsweise zumindest Flächenanteile aus einem semipermeablen Vliesgewebe, wie Tyvek^{®}, bestehen.

Die in der Bestrahlungszone erzeugte Sterilgrenze gewährleistet von hier in Richtung Containment aseptische Verhältnisse, z.B. gemäss Reinraumklasse A nach DIN EN ISO 14644-1. Die Aufrechterhaltung der Sterilgrenze wird bei fortgesetzt fliessender Luftströmung durch die Bestrahlungszone auch bei abgeschalteten Elektronen-Strahlungsquellen gewährleistet.

Die Produktionsanlage zum seriellen Transfer von Objekten durch eine Prozesskammer zur Weiterverarbeitung in einem Containment unter aseptischen Bedingungen ist in den Vorrichtungsansprüchen 15-29 definiert.

### Kurzbeschreibung der beigefügten Zeichnungen

Es zeigen:
- Figur 1: - ein mit dem erfindungsgemässen Verfahren in der zugehörigen Produktionsanlage zu verarbeitendem Objekt; Arbeitsphase 1 (Startsituation)
- Figur 2: - Arbeitsphase 2;
- Figur 3: - Arbeitsphase 3;
- Figur 4: - Arbeitsphase 4;
- Figur 5: - Arbeitsphase 5;
- Figur 6: - Arbeitsphase 6;
- Figur 7: - Arbeitsphase 7;
- Figur8: - Arbeitsphase 8;
- Figur 9: - Arbeitsphase 9;
- Figur 10: - Arbeitsphase 10;
- Figur 11: - Arbeitsphase 11;
- Figur 12: - Arbeitsphase 12;
- Figur 13: - Arbeitsphase 13; und
- Figur 14: - Arbeitsphase 14.

Mit Bezug auf die beiliegenden Zeichnungen erfolgt nachstehend die detaillierte Beschreibung des erfindungsgemässen Verfahrens mit der dazu konzipierten Produktionsanlage zum seriellen Transfer von Objekten durch eine Prozesskammer zur Weiterverarbeitung in einem Containment unter aseptischen Bedingungen. Dies mittels, zumindest und vorzugsweise, zweier in der Prozesskammer installierten Elektronen-Strahlungsquellen.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

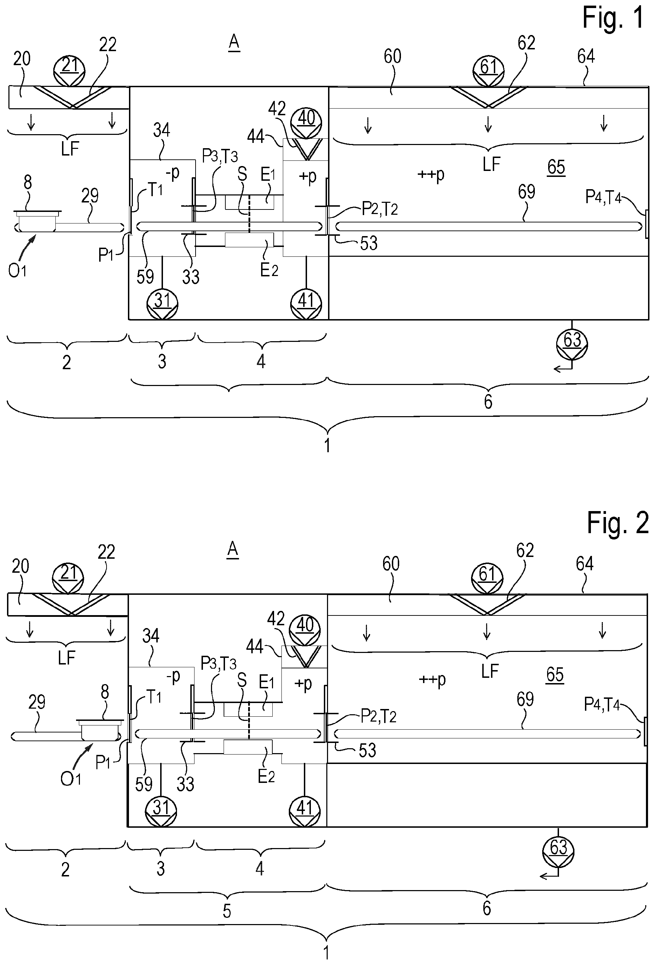

### Figur 1 - Arbeitsphase 1 (Startsituation)

Die Produktionsanlage **1** ist im Aufstellraum **A** mit der Reinraumklasse Grade C positioniert und ist eine Aneinanderkettung von einer Bereitstellungszone **2,** an die sich die Prozesskammer **5** anschliesst, der das Containment **6** folgt. Die Prozesskammer **5** gliedert sich in eine an die Bereitstellungszone **2** direkt anschliessende Vorkammer **3** und eine sich an die Vorkammer **3** anschliessende Bestrahlungszone **4,** von der es in das Containment **6** übergeht. In der Startsituation wird davon ausgegangen, dass die im Containment **6** von dessen Gehäuse **64** umgebene Isolatorkammer **65** und die Prozesskammer **5** dekontaminiert wurden, z.B. herkömmlich mit verdampftem oder zerstäubtem H₂O₂. Die Bereitstellungszone **2** wird mit Laminar Flow **LF** bestrahlt, welcher als Luftstrom aus dem Aufstellraum **A** von einem Zuluftventilator **21** angesaugt und durch einen im Plenum **20** installierten Zuluftfilter **22** gereinigt, erzeugt wird. Für den Transport der Objekte **O1-Ox** aus der Bereitstellungszone **2** hin zur Vorkammer **3** ist ein erstes Transfermittel **29,** z.B. ein Förderband, vorgesehen. In der hier gezeigten Startsituation wird ein erstes Objekt **O1** auf dem ersten Transfermittel **29** bereitgestellt.

Das Objekt **O1** ist z.B. ein wannenförmiges Tab und mit der Abdeckung **8,** zumeist eine versiegelte Tyvek^{®}-Folie, verschlossen, so dass der Innenraum des Objekts **O1** sterilen Zustand bewahrt. Im Innenraum sind Artikel, wie Phiolen, Vials oder Spritzen gelagert, die im Containment **6** weiterverarbeitet werden sollen, z.B. mit Pharmaka befüllt werden. Die Vorkammer **3** ist vom Gehäuse **34** umgeben, innerhalb derer ein Unterdruck **-p** herrscht, z.B. -15 Pa, welcher vom Abluftventilator **31** erzeugt wird.

Innerhalb der Prozesskammer **5** verläuft ein zweites Transfermittel **59,** z.B. ein weiteres Förderband, mit dem der Weitertransport des jeweiligen Objekts **O1-Ox** erfolgt. Am Übergang von der Vorkammer **3** in die Bestrahlungszone **4** befindet sich im Gehäuse **34** eine dritte Passage **P3** zum Durchlass der Objekte **O1-Ox**, bei geöffnetem drittem beweglichem Tor **T3.** Dieses dritte bewegliche Tor **T3.** Ist partiell luftdurchlässig und als Separator ausgebildet. An dieser dritten Passage **P3** ist ein kragenartiges erstes Strömungselement **33** vorgesehen, das sich in die Vorkammer **3** und/oder in die Bestrahlungszone **4** erstreckt. Das erste Strömungselement **33** ist komplementär zur Aussenkontur des einzelnen Objekts **O1-Ox** gestaltet, um den Luftdurchlass bei geöffnetem drittem Tor **T3** und darin einfahrendem bzw. daraus ausfahrendem Objekt **O1-Ox** zu reduzieren.

In der vom Gehäuse **44** umgebenen Bestrahlungszone **4** sind zwei ein- und ausschaltbare Elektronen-Strahlungsquellen **E1,E2** installiert, zwischen denen das jeweilige Objekt **O1-Ox** zur Erzielung seiner äusserlichen Keimfreiheit hindurchgeführt wird. Der in der Bestrahlungszone **4** installierte Zuluftventilator **40** mit zugehörigem Zuluftfilter **42** sowie der Abluftventilator **41** erzeugen in der Bestrahlungszone **4** einen geringen Überdruck **+p,** z.B. +30 Pa.

Durch eine kontinuierliche Luftführung in der Prozesskammer **5** wird eine Sterilgrenze **S** im Wirkungsbereich der von den Elektronen-Strahlungsquellen **E1,E2** gebildeten Bestrahlungszone **4** gewahrt, wobei erst ab dieser Sterilgrenze **S** in Richtung Containment **6** aseptische Verhältnisse herrschen. Die zwei Elektronen-Strahlungsquellen **E1,E2** werden nur für eine Zeitdauer **t** zur äusserlichen Bestrahlung des einzelnen dazwischen hindurchgeführten Objekts **O1-Ox** eingeschaltet, ansonsten sind diese ausgeschaltet.

Am Übergang von der Bestrahlungszone **4** zum Containment **6** ist eine zweite Passage **P2** zum Durchlass der Objekte **O1-Ox** bei geöffnetem zweitem beweglichem, strahlungsabschirmendem Tor **T2,** vorgesehen. An der zweiten Passage **P2** ist ein zweites kragenartiges Strömungselement **53** vorgesehen, das sich in die Bestrahlungszone 4 und/oder in das Containment **6** erstreckt. Auch dieses zweite Strömungselement **53** ist komplementär zur Aussenkontur des einzelnen Objekts **O1-Ox** gestaltet, um den Luftdurchlass bei geöffnetem zweitem Tor **T2** und darin einfahrendem bzw. daraus ausfahrendem Objekt **O1-Ox** zu reduzieren.

Innerhalb des Containments **6** erstreckt sich ein drittes Transfermittel **69,** z.B. ein nächstes Förderband, mit dem der Weitertransport des jeweiligen Objekts **O1-Ox** durch das Containment **6** erfolgt, z.B. nach Verarbeitung der Objekte **O1-Ox** bis an die vierte Passage **P4** mit dem vierten beweglichem Tor **T2** zum Ausschleusen der Objekte **O1-Ox** aus der Isolatorkammer **65.** Mit dem im Plenum **60** des Containments **6** installierten Zuluftventilator **61** mit zugehörigem Zuluftfilter **62** sowie dem einstellbaren Abluftventilator **63** wird ein das Containment **6** durchfliessender gereinigter Luftstrom **LF** erzeugt, wobei ein erhöhter Überdruck **++p** vorteilhaft ist, z.B. +45 Pa

Während dieser Startsituation:
- ist die Produktionsanlage **1** anfahrbereit dekontaminiert, die kontinuierliche Luftführung ist aktiviert und die Sterilgrenze **S** stellt sich ein;
- steht ein erstes Objekt **O1** anfänglich auf dem ersten Transfermittel **29** in der Bereitstellungszone **2;**
- sind die Tore **T1, T2, T3** und **T4** geschlossen; und
- sind die Elektronen-Strahlungsquellen (**E1,E2**) ausgeschaltet.

Im weiteren Produktionsablauf gemäss Arbeitsphasen 2 bis 14 (siehe Figuren 2-14) werden nur die jeweils zur vorherigen Arbeitsphase geänderten Zustände benannt.

### Figur 2 - Arbeitsphase 2

Während dieser Arbeitsphase:
- ist das erste Objekt **O1** auf dem ersten Transfermittel **29** in der Bereitstellungszone **2** bis an die erste Passage **P1** bei weiterhin geschlossenem erstem Tor **T1** herangefahren.

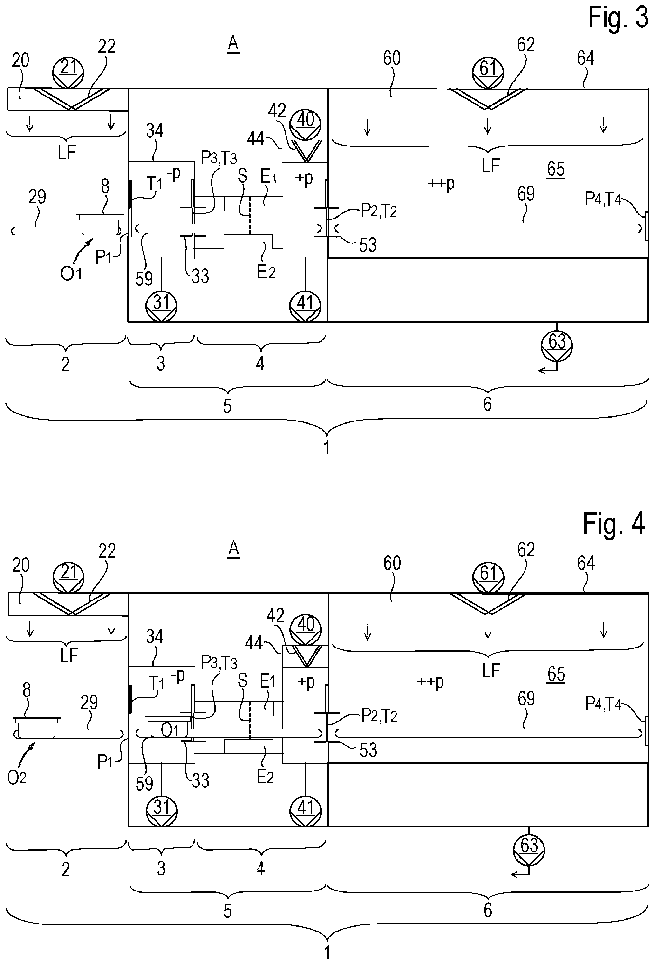

### Figur 3 - Arbeitsphase 3

Während dieser Arbeitsphase:
- wird das erste Tor **T1** geöffnet und somit die erste Passage **P1** freigegeben.

### Figur 4 - Arbeitsphase 4

Während dieser Arbeitsphase:
- ist durch die erste, nun offene Passage **P1** das erste Objekt **O1** auf das zweite Transfermittel **59** geladen und bis in das erste Strömungselement **33** eingefahren, steht jedoch noch vor dem geschlossenen dritten Tor **T3;** und
- wurde ein zweites Objekt **02** anfänglich auf dem ersten Transfermittel **29** in der Bereitstellungszone **2** geladen.

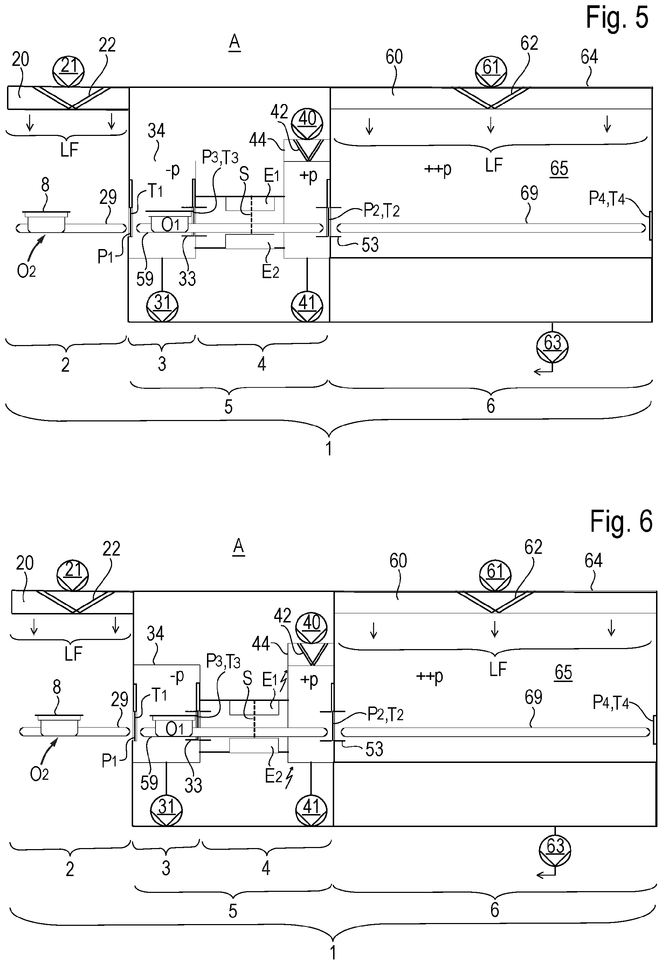

### Figur 5 - Arbeitsphase 5

Während dieser Arbeitsphase:
- rückte das zweite Objekt **O2** auf dem ersten Transfermittel **29** in der Bereitstellungszone **2** in Richtung Prozesskammer **5** vor; und
- wird das erste Tor **T1** geschlossen.

### Figur 6 - Arbeitsphase 6

Während dieser Arbeitsphase:
- rückte das zweite Objekt **O2** auf dem ersten Transfermittel **29** in der Bereitstellungszone **2** weiter in Richtung Prozesskammer **5** vor; und
- werden die zwei Elektronen-Strahlungsquellen **E1,E2** eingeschaltet.

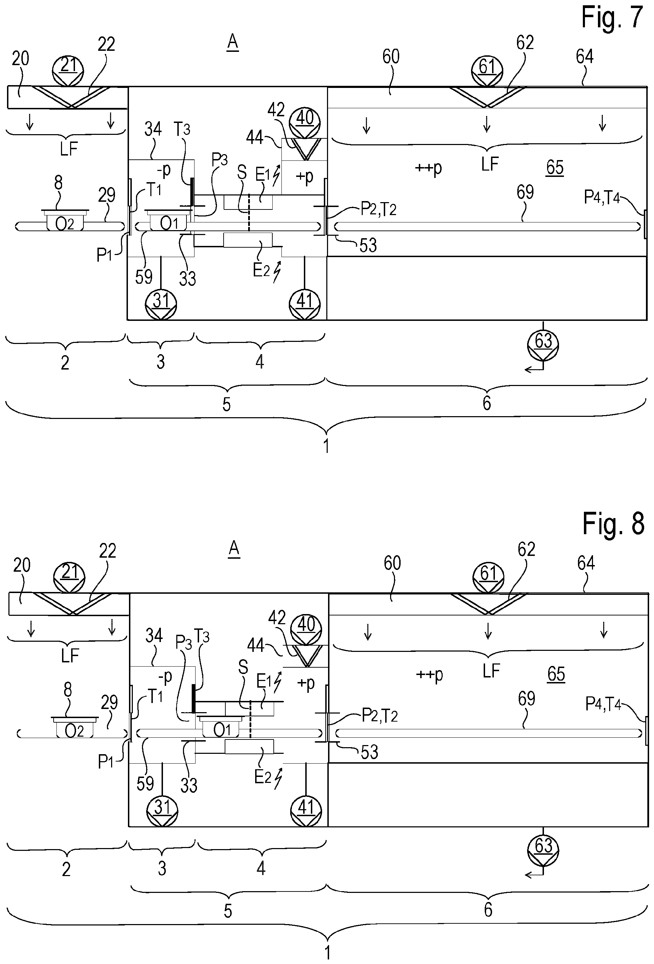

### Figur 7 - Arbeitsphase 7

Während dieser Arbeitsphase:
- rückte das zweite Objekt **O2** auf dem ersten Transfermittel **29** in der Bereitstellungszone **2** erneut in Richtung Prozesskammer **5** vor; und
- das dritte Tor **T3** wird geöffnet, so dass die dritte Passage **P3** offensteht.

### Figur 8 - Arbeitsphase 8

Während dieser Arbeitsphase:
- rückte das zweite Objekt **O2** auf dem ersten Transfermittel **29** in der Bereitstellungszone **2** erneut weiter in Richtung Prozesskammer **5** vor; und
- ist das erste Objekt **O1** auf dem zweiten Transfermittel **59** aus dem ersten Strömungselement **33** ausgefahren und zwischen die aktivierten Elektronen-Strahlungsquellen **E1,E2** eingefahren.

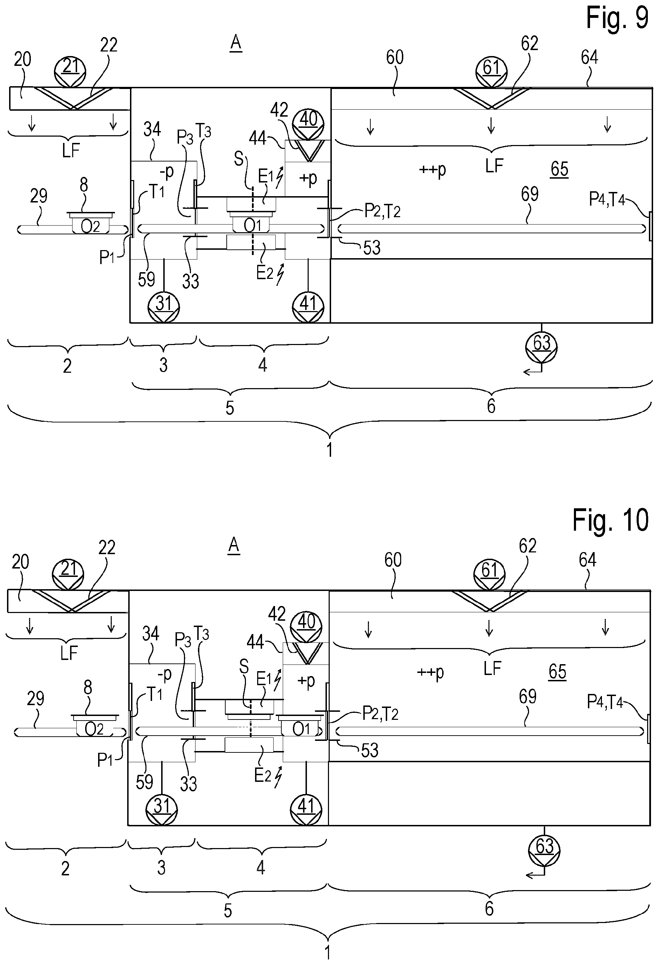

### Figur 9 - Arbeitsphase 9

Während dieser Arbeitsphase:
- rückte das zweite Objekt **O2** auf dem ersten Transfermittel **29** in der Bereitstellungszone **2** wiederum weiter in Richtung Prozesskammer **5** vor;
- ist das erste Objekt **O1** auf dem zweiten Transfermittel **59** vollständig zwischen die aktivierten Elektronen-Strahlungsquellen **E1,E2** eingefahren; und
- das dritte Tor **T3** wird geschlossen.

### Figur 10 - Arbeitsphase 10

Während dieser Arbeitsphase:
- rückte das zweite Objekt **O2** auf dem ersten Transfermittel **29** in der Bereitstellungszone **2** wiederum weiter in Richtung Prozesskammer **5** vor; und
- wurde das erste vollständig bestrahlte Objekt **O1** mittels des zweiten Transfermittels **59** bis in das zweite Strömungselement **53** eingefahren, steht jedoch noch vor dem geschlossenen zweiten Tor **T2.**

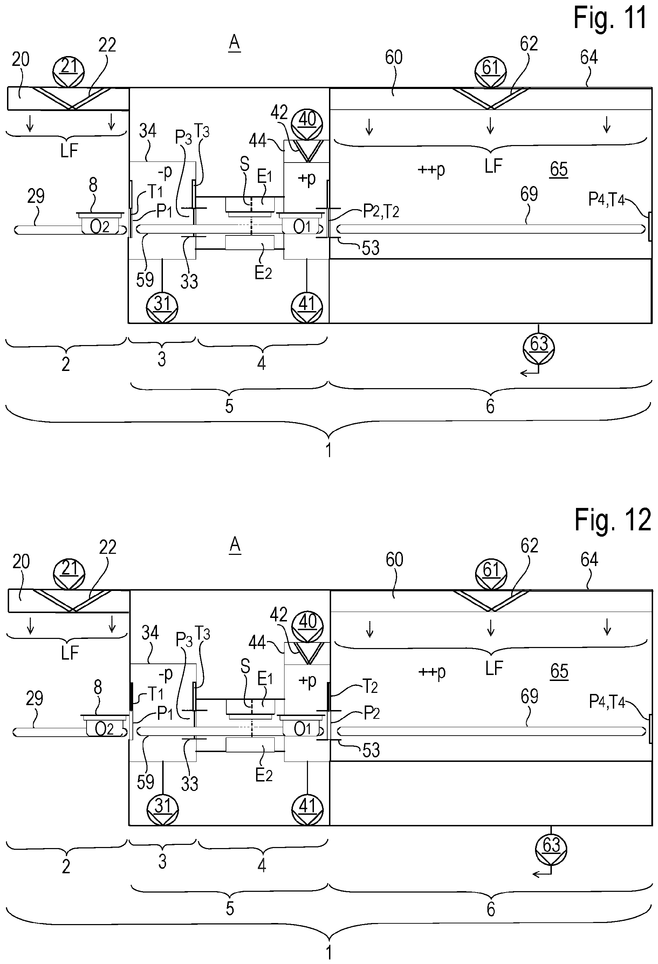

### Figur 11 - Arbeitsphase 11

Während dieser Arbeitsphase:
- rückte das zweite Objekt **O2** auf dem ersten Transfermittel **29** in der Bereitstellungszone 2 immer weiter in Richtung Prozesskammer 5 vor; und
- werden die Elektronen-Strahlungsquellen **E1,E2** abgeschaltet.

### Figur 12 - Arbeitsphase 12

Während dieser Arbeitsphase:
- rückte das zweite Objekt **O2** auf dem ersten Transfermittel **29** in der Bereitstellungszone **2** bis an das jetzt geöffnete erste Tor **T1** heran; und
- das zweite Tor **T2** wird geöffnet, so dass die zweite Passage **T2** zum Durchlass des ersten Objekts **O1** offensteht.

Zum beschleunigten Durchsatz des ersten und zweiten Objekts **O1,O2** können das erste und zweite Tor **T1,T2** gleichzeitig geöffnet und nach Durchlass beider Objekte **01,02** durch die jeweilige Passage **P1,P2** beide Tore **T1,T2** gleichzeitig wieder geschlossen werden.

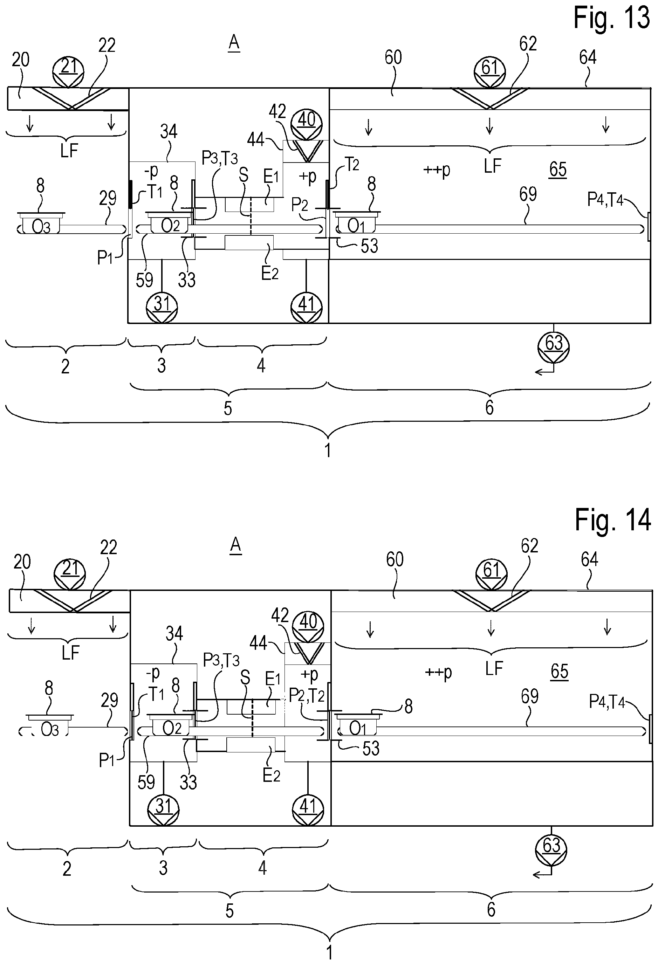

### Figur 13 - Arbeitsphase 13

Während dieser Arbeitsphase:
- das erste Objekt **O1** wurde vom dritten Transfermittel **69** in der Isolatorkammer **65** übernommen, steht jedoch mit seinem hinteren Ende noch im zweiten Strömungselement **53;** und
- ein nächstes, drittes Objekt **03** wird auf das erste Transfermittel **29** in der Bereitstellungszone **2** geladen.

### Figur 14 - Arbeitsphase 14

Während dieser Arbeitsphase:
- rückt das dritte Objekt **O3** auf dem ersten Transfermittel **29** in Richtung Prozesskammer **5** vor; und
- werden das erste Tor **T1** und das zweite Tor **T2** geschlossen.

## Patentansprüche

1. Verfahren zum seriellen Transfer von Objekten (**O1-Ox**) durch eine Prozesskammer (**5**) zur Weiterverarbeitung in einem Containment (**6**) unter aseptischen Bedingungen, wobei:
a) der Prozesskammer (**5**) eine Bereitstellungszone (**2**) vorgelagert ist, aus der das jeweilige Objekt (**O1-Ox**) in die Prozesskammer (**5**) geladen wird, um hier die Oberfläche des jeweiligen Objekts (**O1-Ox**) zu dekontaminieren;
b) die Bereitstellungszone (**2**) mit der Prozesskammer (**5**) und dem sich dran anschliessenden Containment (**6**) eine in einem Aufstellungsraum (**A**) errichtete Produktionsanlage (**1**) bilden;
c) in der Prozesskammer (**5**) zumindest zwei Elektronen-Strahlungsquellen (**E1, E2**) installiert sind, zwischen denen das jeweilige Objekt (**O1-Ox**) zur Erzielung seiner äusserlichen Dekontamination hindurchgeführt wird; und
d) das einzelne Objekt (**O1-Ox**) eine Aussenkontur aufweist, **dadurch gekennzeichnet dass**
e) durch eine kontinuierliche Luftführung in der Prozesskammer (**5**) eine Sterilgrenze (**S**) im Wirkungsbereich einer von den Elektronen-Strahlungsquellen (**E1,E2**) gebildeten Bestrahlungszone (**4**) gewahrt wird, und erst ab dieser Sterilgrenze (**5**) in Richtung Containment (**6**) aseptische Verhältnisse herrschen; und
f) die zumindest zwei Elektronen-Strahlungsquellen (**E1,E2**) nur für eine Zeitdauer (**t**) zur äusserlichen Bestrahlung des einzelnen dazwischen hindurchgeführten Objekts (**O1-Ox**) eingeschaltet werden, ansonsten ausgeschaltet sind.

2. Verfahren nach Anspruch 1, **dadurch gekennzeichnet, dass** die Prozesskammer (**5**) sich in die Bestrahlungszone (**4**) und eine an die Bereitstellungszone (**2**) angrenzende Vorkammer (**3**) gliedert.

3. Verfahren nach Anspruch 2, **dadurch gekennzeichnet, dass**
a) am Übergang von der Bereitstellungszone (**2**) zur Vorkammer (**3**) eine erste Passage (**P1**) zum Durchlass der Objekte (**O1-Ox**), bei einem geöffneten ersten beweglichen, strahlungsabschirmenden Tor (**T1**)**,** vorgesehen ist; und
b) am Übergang von der Bestrahlungszone (**4**) zum Containment **(**6) eine zweite Passage (**P2**) zum Durchlass der Objekte (**O1-Ox**), bei einem geöffneten zweiten beweglichen, strahlungsabschirmenden Tor (**T2**), vorgesehen ist.

4. Verfahren nach zumindest einem der Ansprüche 2 und 3, **dadurch gekennzeichnet, dass**
a) am Übergang von der Vorkammer (**3**) zur Bestrahlungszone **(4)** eine dritte Passage **(**P3**)** zum Durchlass der Objekte (**O1-Ox**), bei einem geöffneten dritten beweglichen Tor **(T3),** vorgesehen ist; und
b) dieses dritte Tor **(T3)** in geschlossener Position bei der Luftführung innerhalb der Prozesskammer **(5)** als Separator partiell luftdurchlässig ist.

5. Verfahren nach Anspruch 4, **dadurch gekennzeichnet, dass** zum beschleunigten Durchsatz des ersten und zweiten Objekts **(O1,O2),** bei geschlossenem dritten Tor **(T3),** das erste und zweite Tor **(T1,T2)** gleichzeitig geöffnet und nach Durchlass beider Objekte (**O1,O2**) durch die jeweilige Passage (**P1,P2**) beide Tore **(T1,T2)** gleichzeitig wieder geschlossen werden können.

6. Verfahren nach zumindest einem der Ansprüche 4 und 5, **dadurch gekennzeichnet, dass**
a) an der dritten Passage **(P3)** ein kragenartiges erstes Strömungselement **(33)** vorgesehen ist, das sich in die Vorkammer **(3)** und/oder in die Bestrahlungszone **(4)** erstreckt; und
b) das erste Strömungselement **(33)** komplementär zur Aussenkontur des einzelnen Objekts (**O1-Ox**) gestaltet ist, um den Luftdurchlass bei geöffnetem drittem Tor **(T3)** und darin einfahrendem bzw. daraus ausfahrendem Objekt (**O1-Ox**) zu reduzieren.

7. Verfahren nach Anspruch 3, **dadurch gekennzeichnet, dass**
a) an der zweiten Passage **(P2)** ein kragenartiges zweites Strömungselement **(53)** vorgesehen ist, das sich in die Bestrahlungszone **(4)** und/oder in das Containment **(6)** erstreckt; und
b) das zweite Strömungselement **(53)** komplementär zur Aussenkontur des einzelnen Objekts (**O1-Ox**) gestaltet ist, um den Luftdurchlass bei geöffnetem zweitem Tor **(T2)** und darin einfahrendem bzw. daraus ausfahrendem Objekt (**O1-Ox**) zu reduzieren.

8. Verfahren nach zumindest einem der Ansprüche 1 bis 7, **dadurch gekennzeichnet, dass** die zumindest zwei Elektronen-Strahlungsquellen **(E1,E2)** im eingeschalteten Betrieb mit unterschiedlicher Beschleunigungsspannung und/oder unterschiedlichem Emissionsstrom betrieben werden, woraus unterschiedliche Strahlencharakteristiken entstehen, so dass die Wirkung der Elektronen-Strahlungsquellen **(E1,E2)** nicht durch eine das Objekt (**O1-Ox**) verschliessende Abdeckung **(8),** schädigend in das Innere des Objekts (**O1-Ox**), gelangt.

9. Verfahren nach zumindest einem der Ansprüche 1 bis 7, **dadurch gekennzeichnet, dass** die zumindest zwei Elektronen-Strahlungsquellen **(E1,E2)** im eingeschalteten Betrieb in unterschiedlichen Abständen zum jeweils zu bestrahlenden Objekt (**O1-Ox**) positioniert sind, so dass die Elektronenstrahlen der zumindest zwei Elektronen-Strahlungsquellen **(E1,E2)** mit unterschiedlicher Strahlencharakteristik auf das jeweils von den Elektronenstrahlen erfasste Oberflächenareal des Objekts (**O1-Ox**) treffen, z.B. damit die Wirkung der Elektronen-Strahlungsquellen **(E1,E2)** nicht durch eine das Objekt (**O1-Ox**) verschliessende Abdeckung **(8),** schädigend in das Innere des Objekts (**O1-Ox**), gelangt.

10. Verfahren nach zumindest einem der Ansprüche 1 bis 9, **dadurch gekennzeichnet, dass** die Zeitdauer **(t)** der zumindest zwei Elektronen-Strahlungsquellen **(E1,E2)** im eingeschalteten Betrieb:
a) vorzugsweise gleichlang und gleichzeitig eingestellt wird; oder
b) gleichzeitig, aber mit ungleicher Länge eingestellt wird; oder
c) gleichlang, aber zeitlich versetzt eingestellt wird; oder
d) mit ungleicher Länge eingestellt wird.

11. Verfahren nach zumindest einem der Ansprüche 1 bis 10, **dadurch gekennzeichnet, dass**
a) das Containment **(6)** von einem aufbereiteten Luftstrom **(LF)** durchströmt wird und im Containment **(6)** ein erhöhter Überdruck **(++p)** herrscht;
b) die Bestrahlungszone **(4)** von einem aufbereiteten Luftstrom **(LF)** durchströmt wird und in der Bestrahlungszone **(4)** ein geringer Überdruck **(+p)** herrscht; und
c) die Vorkammer **(3)** von einem Luftstrom durchströmt wird und in der Vorkammer **(3)** ein Unterdruck **(-p)** herrscht.

12. Verfahren nach zumindest einem der Ansprüche 8 bis 11, **dadurch gekennzeichnet, dass** das einzelne Objekt (**O1-Ox**) ein Behältnis mit einem aseptischen Innenraum aufweist und einen Durchlass hat, der mit der Abdeckung **(8)** verschlossen ist, wobei im Innenraum gelagerte Artikel nach Öffnen der Abdeckung **(8)** zur Behandlung im Containment **(6)** bestimmt sind.

13. Verfahren nach Anspruch 12, **dadurch gekennzeichnet, dass**
a) das Behältnis z.B. ein wannenförmiges Tub oder Tray ist;
b) die Abdeckung (8) typischerweise auf dem den Durchlass des Behältnisses umlaufenden Rand versiegelt ist und so den Innenraum des Behältnisses in sterilem Zustand bewahrt;
c) das Behältnis verschiedene Oberflächenareale aufweist, die sich als Konturen zum Unterfassen oder klemmenden Ergreifen des Objekts (**O1-Ox**) eignen;
d) die Artikel insbesondere Phiolen, Vials, Spritzen oder Karpulen sind; und
e) von der Abdeckung **(8)** vorzugsweise zumindest Flächenanteile aus einem semipermeablen Vliesgewebe, wie Tyvek^{®}, bestehen.

14. Verfahren nach zumindest einem der Ansprüche 1 bis 13, **dadurch gekennzeichnet, dass**
a) die in der Bestrahlungszone **(4)** erzeugte Sterilgrenze **(S),** von hier in Richtung Containment **(6),** aseptische Verhältnisse gewährleistet, z.B. gemäss Reinraumklasse A nach DIN EN ISO 14644-1; und
b) die Aufrechterhaltung der Sterilgrenze **(5)** bei fortgesetzt fliessender Luftströmung durch die Bestrahlungszone **(4)** auch bei abgeschalteten Elektronen-Strahlungsquellen (**E1, E2**) gewährleistet wird.

15. Produktionsanlage **(1)** zum seriellen Transfer von Objekten (**O1-Ox)** durch eine Prozesskammer **(5)** zur Weiterverarbeitung in einem Containment **(6)** unter aseptischen Bedingungen, wobei:
a) der Prozesskammer **(5)** eine Bereitstellungszone **(2)** vorgelagert ist, aus der das jeweilige Objekt (**O1-Ox**) in die Prozesskammer **(5)** zu laden ist, um hier die Oberfläche des jeweiligen Objekts (**O1-Ox**) zu dekontaminieren;
b) die Bereitstellungszone **(2)** mit der Prozesskammer **(5)** und dem sich dran anschliessenden Containment **(6)** eine in einem Aufstellungsraum **(A)** errichtete Produktionsanlage **(1)** bilden;
c) in der Prozesskammer **(5)** zumindest zwei Elektronen-Strahlungsquellen (**E1,E2**) installiert sind, zwischen denen das jeweilige Objekt (**O1-Ox**) zur Erzielung seiner äusserlichen Dekontamination hindurch zu führen ist; und
d) das einzelne Objekt (**O1-Ox**) eine Aussenkontur aufweist, **dadurch gekennzeichnet dass**
e) infolge kontinuierlicher Luftführung in der Prozesskammer **(5)** eine Sterilgrenze **(S)** im Wirkungsbereich einer von den Elektronen-Strahlungsquellen (**E1,E2**) gebildeten Bestrahlungszone **(4)** entsteht, wobei ab dieser Sterilgrenze **(5)** in Richtung Containment **(6)** aseptische Verhältnisse herrschen; und
f) die zumindest zwei Elektronen-Strahlungsquellen **(E1,E2)** nur für eine Zeitdauer **(t)** zur äusserlichen Bestrahlung des einzelnen dazwischen hindurchgeführten Objekts (**O1-Ox**) eingeschaltet werden, ansonsten ausgeschaltet sind.

16. Produktionsanlage **(1)** nach Anspruch 15, **dadurch gekennzeichnet, dass** die Prozesskammer **(5)** sich in die Bestrahlungszone **(4)** und eine an die Bereitstellungszone **(2)** angrenzende Vorkammer **(3)** gliedert.

17. Produktionsanlage **(1)** nach Anspruch 16, **dadurch gekennzeichnet, dass**
a) am Übergang von der Bereitstellungszone **(2)** zur Vorkammer **(3)** eine erste Passage **(P1)** zum Durchlass der Objekte (**O1-Ox**), bei einem geöffneten ersten beweglichen, strahlungsabschirmenden Tor **(T1),** vorgesehen ist; und
b) am Übergang von der Bestrahlungszone **(4)** zum Containment **(6)** eine zweite Passage **(P2)** zum Durchlass der Objekte (**O1-Ox**), bei einem geöffneten zweiten beweglichen, strahlungsabschirmenden Tor **(T2),** vorgesehen ist.

18. Produktionsanlage **(1)** nach zumindest einem der Ansprüche 16 und 17, **dadurch gekennzeichnet, dass**
a) am Übergang von der Vorkammer **(3)** zur Bestrahlungszone **(4)** eine dritte Passage **(P3)** zum Durchlass der Objekte (**O1-Ox**), bei einem geöffneten dritten beweglichen Tor **(T3),** vorgesehen ist; und
b) dieses dritte Tor **(T3)** in geschlossener Position bei der Luftführung innerhalb der Prozesskammer **(5)** als Separator partiell luftdurchlässig ist.

19. Produktionsanlage **(1)** nach Anspruch 18, **dadurch gekennzeichnet, dass** zum beschleunigten Durchsatz des ersten und zweiten Objekts **(01,02),** bei geschlossenem dritten Tor **(T3),** das erste und zweite Tor **(T1,T2)** gleichzeitig geöffnet und nach Durchlass beider Objekte (**O1,O2**) durch die jeweilige Passage **(P1,P2)** beide Tore **(T1,T2)** gleichzeitig wieder geschlossen sind.

20. Produktionsanlage **(1)** nach Anspruch 18, **dadurch gekennzeichnet, dass**
a) an der dritten Passage **(P3)** ein kragenartiges erstes Strömungselement **(33)** vorgesehen ist, das sich in die Vorkammer **(3)** und/oder in die Bestrahlungszone **(4)** erstreckt; und
b) das erste Strömungselement **(33)** komplementär zur Aussenkontur des einzelnen Objekts (**O1-Ox**) gestaltet ist, um den Luftdurchlass bei geöffnetem drittem Tor **(T3)** und darin einfahrendem bzw. daraus ausfahrendem Objekt (**O1-Ox**) zu reduzieren.

21. Produktionsanlage **(1)** nach Anspruch 17, **dadurch gekennzeichnet, dass**
a) an der zweiten Passage **(P2)** ein kragenartiges zweites Strömungselement **(53)** vorgesehen ist, das sich in die Bestrahlungszone **(4)** und/oder in das Containment **(6)** erstreckt; und
b) das zweite Strömungselement **(53)** komplementär zur Aussenkontur des einzelnen Objekts (**O1-Ox**) gestaltet ist, um den Luftdurchlass bei geöffnetem zweitem Tor **(T2)** und darin einfahrendem bzw. daraus ausfahrendem Objekt (**O1-Ox**) zu reduzieren.

22. Produktionsanlage **(1)** nach zumindest einem der Ansprüche 15 bis 21, **dadurch gekennzeichnet, dass** die zumindest zwei Elektronen-Strahlungsquellen **(E1,E2)** im eingeschalteten Betrieb mit unterschiedlicher Beschleunigungsspannung und/oder unterschiedlichem Emissionsstrom betrieben werden, woraus unterschiedliche Strahlencharakteristiken entstehen, so dass die Wirkung der Elektronen-Strahlungsquellen **(E1,E2)** nicht durch eine das Objekt (**O1-Ox**) verschliessende Abdeckung **(8),** schädigend in das Innere des Objekts (**O1-Ox**), gelangt.

23. Produktionsanlage **(1)** nach zumindest einem der Ansprüche 15 bis 21, **dadurch gekennzeichnet, dass** die zumindest zwei Elektronen-Strahlungsquellen **(E1,E2)** im eingeschalteten Betrieb in unterschiedlichen Abständen zum jeweils zu bestrahlenden Objekt (**O1-Ox**) positioniert sind, so dass die Elektronenstrahlen der zumindest zwei Elektronen-Strahlungsquellen **(E1,E2)** mit unterschiedlicher Strahlencharakteristik auf das jeweils von den Elektronenstrahlen erfasste Oberflächenareal des Objekts (**O1-Ox**) treffen, z.B. damit die Wirkung der Elektronen-Strahlungsquellen **(E1,E2)** nicht durch eine das Objekt (**O1-Ox**) verschliessende Abdeckung **(8),** schädigend in das Innere des Objekts (**O1-Ox**), gelangt.

24. Produktionsanlage **(1)** zumindest einem der Ansprüche 15 bis 23, **dadurch gekennzeichnet, dass** die Zeitdauer **(t)** der zumindest zwei Elektronen-Strahlungsquellen **(E1,E2)** im eingeschalteten Betrieb:
a) vorzugsweise gleichlang und gleichzeitig eingestellt ist; oder
b) gleichzeitig, aber mit ungleicher Länge eingestellt ist; oder
c) gleichlang, aber zeitlich versetzt eingestellt ist; oder
d) mit ungleicher Länge eingestellt ist.

25. Produktionsanlage **(1)** nach zumindest einem der Ansprüche 15 bis 24, **dadurch gekennzeichnet, dass**
a) das Containment **(6)** ein aufbereiteter Luftstrom **(LF)** durchströmt und im Containment **(6)** ein erhöhter Überdruck **(++p)** herrscht;
b) die Bestrahlungszone **(4)** ein aufbereiteter Luftstrom **(LF)** durchströmt und in der Bestrahlungszone **(4)** ein geringer Überdruck **(+p)** herrscht; und
c) die Vorkammer **(3)** ein Luftstrom durchströmt und in der Vorkammer **(3)** ein Unterdruck **(-p)** herrscht.

26. Produktionsanlage **(1)** nach zumindest einem der Ansprüche 22 bis 25, **dadurch gekennzeichnet, dass** das einzelne Objekt (**O1-Ox**) ein Behältnis mit einem aseptischen Innenraum aufweist und einen Durchlass hat, der mit der Abdeckung **(8)** verschlossen ist, wobei im Innenraum gelagerte Artikel nach Öffnen der Abdeckung **(8)** zur Behandlung im Containment **(6)** bestimmt sind.

27. Produktionsanlage **(1)** nach Anspruch 26, **dadurch gekennzeichnet, dass**
a) das Behältnis z.B. ein wannenförmiges Tub oder Tray ist;
b) die Abdeckung **(8)** typischerweise auf dem den Durchlass des Behältnisses umlaufenden Rand versiegelt ist und so den Innenraum des Behältnisses in sterilem Zustand bewahrt;
c) das Behältnis verschiedene Oberflächenareale aufweist, die sich als Konturen zum Unterfassen oder klemmenden Ergreifen des Objekts (**O1-Ox**) eignen;
d) die Artikel insbesondere Phiolen, Vials, Spritzen oder Karpulen sind; und
e) von der Abdeckung **(8)** vorzugsweise zumindest Flächenanteile aus einem semipermeablen Vliesgewebe, wie Tyvek , bestehen.

28. Produktionsanlage **(1)** nach zumindest einem der Ansprüche 15 bis 27, **dadurch gekennzeichnet, dass**
a) die in der Bestrahlungszone **(4)** erzeugte Sterilgrenze **(S),** von hier in Richtung Containment **(6),** aseptische Verhältnisse gewährleistet, z.B. gemäss Reinraumklasse A nach DIN EN ISO 14644-1; und
b) die Aufrechterhaltung der Sterilgrenze **(5)** bei fortgesetzt fliessender Luftströmung durch die Bestrahlungszone **(4)** auch bei abgeschalteten Elektronen-Strahlungsquellen **(E1,E2)** gewährleistet wird.

29. Produktionsanlage **(1)** nach zumindest einem der Ansprüche 15 bis 28, **dadurch gekennzeichnet, dass** für den Transport der Objekte (**O1-Ox**) innerhalb der Bereitstellungszone **(2),** innerhalb der Prozesskammer **(5)** und innerhalb des Containments **(6)** sind Transfermittel **(29,59,69)** installiert.
